# EUROPÄISCHE PATENTSCHRIFT

(11) **EP 1 834 595 B1**
(45) Veröffentlichungstag und Bekanntmachung des Hinweises auf die Patenterteilung: **18.05.2011**
(21) Anmeldenummer: 07005105.7
(22) Anmeldetag: 13.03.2007
(51) Int. Cl.: A61B 17/32

(54) **Flexible Hohlwelle für ein medizinisches Instrument**
Flexible quill shaft for a medical instrument
Arbre creux flexible pour un instrument médical

(30) Priorität: 15.03.2006 DE 102006013979
(43) Veröffentlichungstag der Anmeldung: 19.09.2007
(73) Patentinhaber: Karl Storz GmbH & Co. KG, 78532 Tuttlingen (DE)
(72) Erfinder: Efinger, Andreas, 78604 Rietheim (DE); Hermle, Rainer, 78559 Gosheim (DE)
(74) Vertreter: Weller, Wolfgang

(56) Entgegenhaltungen:
- EP-A- 1 382 307
- EP-A- 1 598 023
- US-A- 5 690 660
- US-A1- 2002 029 055
- US-A1- 2002 038 129
- US-A1- 2005 177 168

## Beschreibung

Die Erfindung betrifft ein medizinisches Instrument mit einem gekrümmten Schaft, in dem eine flexible Hohlwelle aufgenommen ist, wobei die flexible Hohlwelle ein proximales Ende zum Koppeln mit einem Antrieb des medizinischen Instrumentes und ein distales Ende aufweist, an dem ein Werkzeug angeordnet ist, wobei die Hohlwelle eine Wandung mit Einschnitten aufweist, so dass die Hohlwelle auch in gekrümmtem Zustand Drehkräfte übertragen kann.

Ein derartiges medizinisches Instrument ist aus der US 2005/0177168 A1 bekannt. In einem Ausführungsbeispiel ist in die flexible Hohlwelle distalseitig ein Schaft eingesteckt, der ein Werkzeug trägt. Proximalseitig ist ein Kuppelfutter eingesteckt, das zur Verbindung mit einem Drehantrieb dient. In einem weiteren Ausführungsbeispiel ist das Werkzeug mit einem schaft- oder drahtförmigen Körper verbunden, der durch den gekrümmten Schaft hindurchreicht und proximalseitig mit einem Antrieb verbunden ist. Im Bereich der Krümmung des Schaftes ist der Körper als relativ dünner Körper aus Federdraht ausgebildet, so dass er in diesem gekrümmten Bereich die entsprechende Krümmung einnehmen kann.

Aus der US 2002/0029055 A1 ist ein medizinisches Instrument in Form eines chirurgischen Bohrers bekannt. Der flexible Bohrer weist einen flexiblen Schaft auf, an dessen distalem Ende ein Werkzeug zum Bohren angeordnet ist. Ein proximales Ende des flexiblen Schaftes ist durch ein Gehäuse mit einem Antrieb verbunden. Im Innern des flexiblen Schafts ist ein flexibler Bohrerstab angeordnet, der mit dem Werkzeug verbunden ist.

Aufgrund der Flexibilität bzw. der Biegsamkeit der Hohlwelle kann das Instrument bzw. dessen Schaft gekrümmt bzw. gebogen ausgebildet sein. Die flexible Hohlwelle ist in dem gekrümmten Schaft aufgenommen und ist proximalseitig mit einem Antrieb verbunden, der die Hohlwelle im gekrümmten Schaft dreht. Am distalen Ende ist die Hohlwelle mit einem Werkzeug, beispielsweise mit einer Schneide oder mit einem Fräskopf, versehen. Das distale Ende der Hohlwelle kommt frei drehbeweglich im distalen Endbereich des gekrümmten Schaftes zum Liegen.

Bei der EP 0 986 989 A1 ist das Werkzeug als eine Schneide ausgebildet, die zum Abtragen von Gewebe dient.

Aus der DE 101 07 156 A1 ist ein medizinisches Instrument bekannt, bei dem das Werkzeug als Fräskopf ausgebildet ist, der über das distale Ende des gekrümmten Schaftes hinausragt.

Die Wandung der Hohlwelle ist mit zahlreichen Einschnitten versehen, durch die die Flexibilität erreicht wird. Diese Öffnungen bestehen beispielsweise aus mäanderförmigen Einschnitten in der Wandung, die längs einer schraubenlinienförmigen Linie verlaufen. Dadurch ist es möglich, dass bei gekrümmter und sich drehender Hohlwelle jeweils an der Außenseite der Krümmung die Einschnitte etwas aufweiten können.

Im praktischen Einsatz wirken auf das Werkzeug Zug- und/oder Druckkräfte ein.

Bei einem Fräskopf, wie er beispielsweise bei Stirnhöhleneingriffen eingesetzt wird, treten Druckkräfte auf, wenn der Fräskopf auf eine Knochenstelle trifft und der Operateur das Instrument kräftig gegen diese Knochenstelle vorschiebt. Zugkräfte können dadurch entstehen, wenn sich der Fräskopf in den Knochen eingefressen hat und man versucht, das Instrument abzuziehen, so dass sich der Fräskopf mit dem Knochen verhakt.

Ähnliche Druck-/Zugbelastungen treten bei Schneiden auf, die sich mit Gewebe oder Knorpel verhaken.

Im praktischen Einsatz wurde nun festgestellt, dass die flexible Hohlwelle Zug- und/oder Druckbelastungen aufgrund ihrer Konstruktion dahingehend folgen kann, dass die Hohlwelle etwas gedehnt oder gestaucht werden kann. In diesen Zuständen läuft die Hohlwelle aber nicht mehr rund sondern baucht etwas aus und schlägt gegen die Innenseite des gekrümmten Schaftes, wodurch unerwünschte Vibrationen entstehen, die die Handhabung des medizinischen Instrumentes erschweren.

Es ist daher Aufgabe der Erfindung, hier Abhilfe zu schaffen und Maßnahmen zu ergreifen, die ein solches unerwünschtes Vibrieren oder Ausschlagen der Hohlwelle verhindern.

Erfindungsgemäß wird die Aufgabe dadurch gelöst, dass am distalen Ende der Hohlwelle ein Anschlag vorgesehen ist, der mit einem Anschlag am Shaft derart zusammenwirkt, dass ein Stauchen der Hohlwelle über ein bestimmtes Maß gesperrt ist, und dass sich in der Hohlwelle ein flexibler, in axialer Richtung jedoch undehnbarer und unstauchbarer Draht vom distalen zum proximalen Ende erstreckt.

Diese Maßnahme hat den Vorteil, dass durch diesen im Inneren der Hohlwelle angeordneten Draht einem Dehnen durch Zugkräfte entgegengewirkt wird und somit eine Zugentlastung bewirkt wird. Dadurch, dass dieser Draht flexibel ausgestattet ist, kann er der Krümmung der Hohlwelle folgen. Durch die Ausbildung als unstauchbarer Draht wird bis zu einem gewissen Maße auch eine Druckentlastung geschaffen, die aber aufgrund der Flexibilität des Drahtes und aufgrund des gekrümmten Zustandes nur in einem bestimmten Maß wirkt, aber dennoch vorhanden ist.

Die Aufgabe wird im Sinne einer Druckentlastung dadurch gelöst dass am distalen Ende ein Anschlag vorgesehen ist, der mit einem Anschlag am Schaft derart zusammenwirkt, dass ein Stauchen der Hohlwelle über ein bestimmtes Maß gesperrt ist.

Diese Maßnahme hat den Vorteil, dass eine Druckentlastung dahingehend geschaffen wird, dass der Anschlag mit einem Anschlag am medizinischen Instrument in Wirkverbindung steht, so dass ein Stauchen der Hohlwelle gesperrt ist und die Druckkräfte über den Anschlag am distalen Ende der Hohlwelle auf den Anschlag am medizinischen Instrument übertragen werden.

Zusätzlich zu dem Anschlag ist in der Hohlwelle ein flexibler, in axialer Richtung jedoch undehnbarer und unstauchbarer Draht vorhanden.

Diese Maßnahme in Kombination mit dem Anschlag sorgt nun sowohl für eine ausreichende Zug- als auch für eine Druckentlastung.

Je nachdem, welchen Belastungen die Hohlwelle beim praktischen Einsatz ausgesetzt ist, liegt es im Rahmen der Erfindung, die Zugentlastung über den undehnbaren und unstauchbaren Draht im Inneren zu bewerkstelligen, der auch eine gewisse Druckentlastung bewirkt. Da beide Entlastungen erwünscht werden, ist die Kombination von Anschlag und undehnbarem und unstauchbarem Körper vorzusehen.

Diese Maßnahme hat den Vorteil, dass durch den Draht zunächst ein sehr schlankes Element zur Verfügung gestellt wird, das im Inneren einer auch sehr dünnen Hohlwelle aufgenommen werden kann. Ein solcher Draht besitzt eine ausreichende Flexibilität, um den Bewegungen der Hohlwelle folgen zu können. Zugleich ist das Drahtmaterial ausreichend fest, um für eine Zugentlastung zu sorgen, da ein solcher Draht starken Zugkräften ohne Verformung im Sinne einer Längendehnung widerstehen kann.

In einer weiteren Ausgestaltung der Erfindung ist der Anschlag am distalen Ende an einer drehbar auf der Hohlwelle angebrachten Hülse angeordnet.

Diese Maßnahme hat den Vorteil, dass die Hülse, wenn deren Anschlag mit dem Anschlag am Instrument zum Liegen kommt, dann ortsfest stehen bleibt und entsprechend die Druckkräfte übertragen kann, während sich die Hohlwelle nach wie vor in der Hülse drehen kann, so dass die Funktionsfähigkeit auch in einem solchen Druckentlastungsfall voll erhalten bleibt.

In einer weiteren Ausgestaltung der Erfindung ist der Anschlag als eine Schulter ausgebildet.

Diese Maßnahme hat den Vorteil, dass auch bei sehr kleinen Bauteilen mit Durchmessern von wenigen Millimetern durch eine solche Schulter eine ausreichend große Kraftübertragungsfläche vorhanden ist, um eine effektive Druckentlastung zu bewerkstelligen.

In einer weiteren Ausgestaltung der Erfindung ist die Hülse axial beweglich am distalen Ende angebracht.

Diese Maßnahme hat den Vorteil, dass ein gewisses axiales Spiel der Hülse vorhanden ist, diese also im Normalbetrieb, wenn kein Druckentlastungsfall vorliegt, lose im distalen Endbereich des gekrümmten Schaftes aufgenommen ist und frei drehbar um die Hohlwelle ist, dass jedoch nach einer gewissen axialen Verschiebung der Anschlag wirkt. Dies eröffnet auch die Möglichkeit, je nach Länge der Hohlwelle ein gewisses Stauchmaß zuzulassen und erst nach Überschreiten dieses zulässigen Stauchmaßes den Anschlag zum Eingriff kommen zu lassen.

In einer weiteren Ausgestaltung der Erfindung ist das Werkzeug am distalen Ende über den Draht mit dem proximalen Ende verbunden.

Diese Maßnahme hat den Vorteil, dass mit wenigen Bauteilen die Zug- und/oder Druckentlastung bewerkstelligt werden kann, indem nämlich der Draht sich zwischen dem Werkzeug am distalen Ende und dem proximalen Ende erstreckt.

In einer weiteren Ausgestaltung der Erfindung sind die Einschnitte der Hohlwelle als schraubenlinienförmig gewundener Schlitz ausgebildet.

Diese an sich bekannte Maßnahme hat den Vorteil, dass eine besonders flexible Hohlwelle geschaffen ist.

In einer weiteren Ausgestaltung der Erfindung ist der schraubenlinienförmig gewundene Schlitz als mäanderförmiger Schlitz ausgebildet.

Diese ebenfalls an sich bekannte Maßnahme hat den Vorteil, dass die Hohlwelle, auch wenn sie relativ lang und dünn ist, hervorragend Drehkräfte übertragen kann. Eine solche Hohlwelle kann relativ leicht auseinandergezogen werden, so dass insbesondere bei solchen mäanderförmigen Schlitzen die Zugentlastung erforderlich und besonders wirksam ist.

In einer weiteren Ausgestaltung der Erfindung sind Abstandhalter vorhanden, die den Draht im Inneren der Hohlwelle in Abstand zu deren Wandung halten.

Diese Maßnahme hat den Vorteil, dass der Draht in Abstand zur Innenseite der Hohlwelle gehalten wird, so dass beispielsweise bei hohen Drehzahlen und gekrümmter Hohlwelle kein Schlagen oder ein unrunder Lauf erfolgt.

In einer weiteren Ausgestaltung der Erfindung sind die Abstandhalter derart ausgestaltet, dass Medien im Inneren der Hohlwelle in axialer Richtung transportierbar sind.

Beim Einsatz der Hohlwellen wird deren Innenraum dazu ausgenutzt, um Medien, beispielsweise eine Spülflüssigkeit, zum proximalen Ende zuzuführen. Es ist auch gebräuchlich, durch das Innere der Hohlwelle von der Operationsstelle Feststoffe, beispielsweise Gewebeteile oder Knochenpartikel, oder Flüssigkeiten wie Blut oder Spülflüssigkeit, über die Hohlwelle abzusaugen. Die Abstandhalter sind so ausgestaltet, dass ein solcher Fluss möglich ist. Dazu sind die Abstandhalter entweder mit entsprechenden Öffnungen versehen, oder sie sind so ausgestaltet, dass sie radial vorstehende Beine haben, zwischen denen ausreichend Öffnungen zur Strömung von solchen Medien vorhanden sind. Darüber hinaus sind diese so ausgestaltet, dass dadurch keine unnötig hohen Strömungswiderstände aufgebaut werden.

In einer weiteren Ausgestaltung der Erfindung ist der Draht und/oder die innere Wandung der Hohlwelle mit einem reibungsgünstigen Material versehen.

Dies kann nun dadurch erfolgen, dass diese Bauteile an sich schon aus einem reibungsarmen Material hergestellt sind oder nachträglich mit einem solchen Material beschichtet sind, beispielsweise mit Teflon.

Diese Maßnahme hat den Vorteil, dass, falls eine Berührung zwischen Draht und Innenseite der Wandung der Hohlwelle in stark gekrümmten Positionen stattfinden sollte, diese reibungsarm abläuft.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.
Es zeigen:
- Fig. 1: eine Seitenansicht einer erfindungsgemäßen flexiblen Hohlwelle in geradlinig ausgestrecktem Zustand,
- Fig. 2: einen stark vergrößerten Längsschnitt der Hohlwelle von Fig. 1,
- Fig. 3: einen Querschnitt einer Hohlwelle mit auf dem Draht aufgebrachtem Abstandhalter in einer ersten Ausführungsform, und
- Fig. 4: eine dem Schnitt von Fig. 3 vergleichbare Darstellung mit einer weiteren Ausgestaltung eines Abstandhalters.

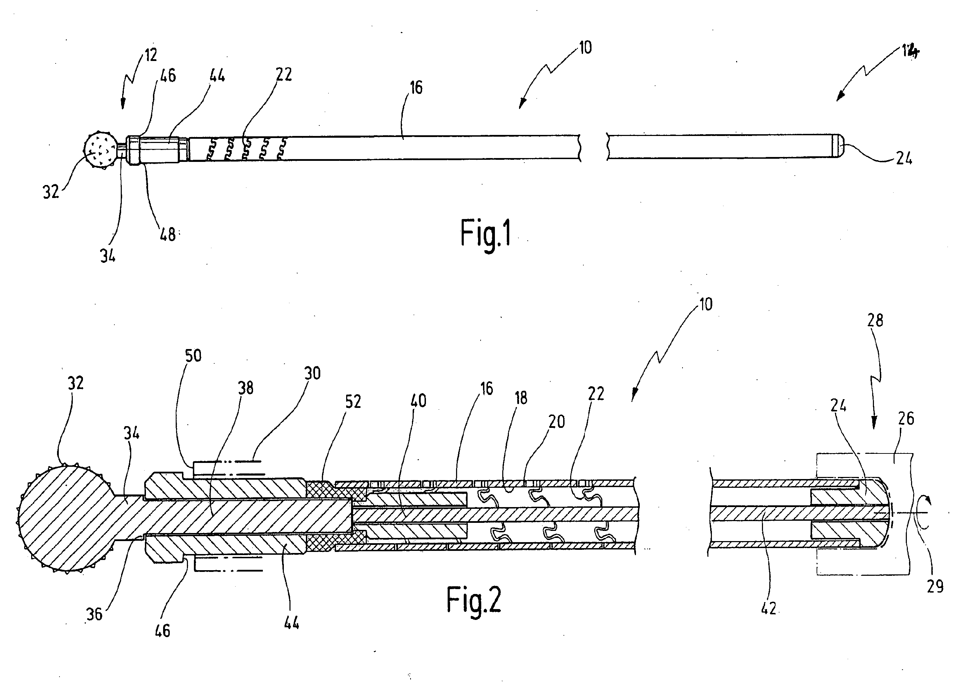

Eine in den Fig. 1 und 2 dargestellte Hohlwelle ist in ihrer Gesamtheit mit der Bezugsziffer 10 bezeichnet.

Die Hohlwelle 10 weist ein distales Ende 12 und ein proximales Ende 14 auf. Im Bereich zwischen distalem Ende 12 und proximalem Ende 14 ist der Körper der Hohlwelle 10 als ein Rohr 16 ausgebildet, in dessen Wandung 18 zahlreiche Einschnitte 20 vorhanden sind.

Die Einschnitte 20 bestehen aus einem durchgehend sich entlang einer Schraubenlinie windenden Mäander 22. In der Darstellung von Fig. 1 ist nur ein Teilabschnitt des Mäanders 22 dargestellt.

Am proximalen Ende 14 ist das Rohr mit einer Kupplung 24 verbunden, die dazu dient, mit einem Antrieb 26 eines medizinischen Instrumentes 28 verbunden zu werden. Über den Antrieb 26 kann die Hohlwelle 10 um deren Längsachse 29 gedreht werden, wie das durch einen Pfeil angedeutet ist.

Die Hohlwelle 10 selbst ist in einem gekrümmten Schaft 30 des medizinischen Instrumentes 28 aufgenommen.

In den Darstellungen der Figuren ist die Hohlwelle 10 in ihrem geradlinig gestreckten Zustand dargestellt, aufgrund des Mäanders 22 weist sie jedoch eine solche Flexibilität auf, dass sie in einem gekrümmten oder bogenförmigen Schaft aufgenommen werden kann und durch den Antrieb 26 in dem gekrümmten Schaft 30 gedreht werden kann.

Das distale Ende 12 ist als ein Werkzeug ausgebildet, im dargestellten Ausführungsbeispiel ein kugeliger Fräskopf 32.

Der Fräskopf 32 geht über einen Hals 34 und über einen Absatz 36 in einen Zapfen 38 über, dessen proximales Ende mit einem Körper 40 verbunden ist, der sich über die gesamte Länge des Rohres 16 bis zum proximalen Ende 14 erstreckt und dort mit der Kupplung 24 fest verbunden ist.

Der Körper 40 ist als eine Drahtlitze bzw. als Draht 42 ausgestaltet, der somit einerseits mit dem Zapfen 38 fest verbunden ist und andererseits mit der Kupplung 24.

Der Draht ist aus einem metallischen Material hergestellt, ist somit flexibel und kann den Krümmungsbewegungen der Hohlwelle 10 folgen. Aufgrund seiner Ausbildung aus metallischem Material ist der Draht 42 zugfest, bildet also eine Zugentlastung.

In anderen Worten ausgedrückt, kann die Hohlwelle 10 nicht mehr gedehnt werden als in dem in Fig. 1 und 2 gezeigten Zustand.

Ohne den mittigen Draht 42 wäre dies in einem relativ großen Maße möglich, und zwar aufgrund des durchgehenden schraubenlinienförmig gewundenen Mäanders 22.

Im praktischen Einsatz ist die Hohlwelle 10 an ihrem proximalen Ende 14 fest mit dem Antrieb 26 verbunden, somit bezüglich ihrer axialen Lage also ortsfest, sie kann sich lediglich drehen.

Zugkräfte, beispielsweise wenn sich der Fräskopf 32 bei einem Eingriff mit einem Knochen verhakt, führen nicht zu einer Längsdehnung der Hohlwelle 10, dies wird durch die Zugentlastung mittels des inneren Drahtes 42 verhindert.

Bis zu einem gewissen Maß kann der Draht 42 auch für eine Druckentlastung sorgen. Wenn allerdings die Hohlwelle sehr dünn ist, ist der Draht ebenfalls sehr dünn, und kann, insbesondere wenn er in dem gekrümmten Schaft 30 aufgenommen ist, nur einen geringen Widerstand gegen Druckkräfte entgegensetzen, da er selbst zum Ausbauchen neigt.

Daher ist um den Zapfen 38 drehbar eine Hülse 44 aufgenommen. Das distale Ende der Hülse 44 liegt an dem Absatz 36 des Zapfens 38 an. An ihrer Außenseite 44 ist die Hülse 44 mit einer Schulter 46 versehen, die als Anschlag 48 wirkt, und zwar wenn diese Schulter 46 auf die entsprechende Stirnkante 50 des Schaftes 30 trifft.

Kommt die Schulter 46 an dieser Stirnkante 50 zum Liegen, kann die Hohlwelle 10 nicht weiter gestaucht werden, d.h. Druckkräfte, die über den Fräskopf 32 auf die Hohlwelle 10 einwirken, werden über den Anschlag 48 auf den gekrümmten Schaft 30 abgeleitet. Somit wird eine Druckentlastung der Hohlwelle 10 bewerkstelligt. Aufgrund der Tatsache, dass die Hülse 44 relativ zum Zapfen 80 drehbar angeordnet ist, kann sich die Hohlwelle 10 in der Hülse 44 drehen, wenn sie im Druckentlastungsfall über ihre Schulter 46 an der Stirnkante 50 zum Liegen kommt und dann dort ortsfest sitzt.

Ein Verbindungsstück 52 sorgt für eine feste Verbindung zwischen Rohr 16 der Hohlwelle 10 und dem Zapfen 38 des Fräskopfes 32.

Bei starken Krümmungen kann eine Berührung zwischen der Innenseite der Hohlwelle 10 und dem Draht 42 erfolgen. Um diese möglichst reibungsarm ablaufen zu lassen, können diese Bauteile mit reibungsgünstigen Materialien, z.B. mit Teflon, beschichtet sein, oder von vornherein aus reibungsgünstigen Materialien hergestellt sein.

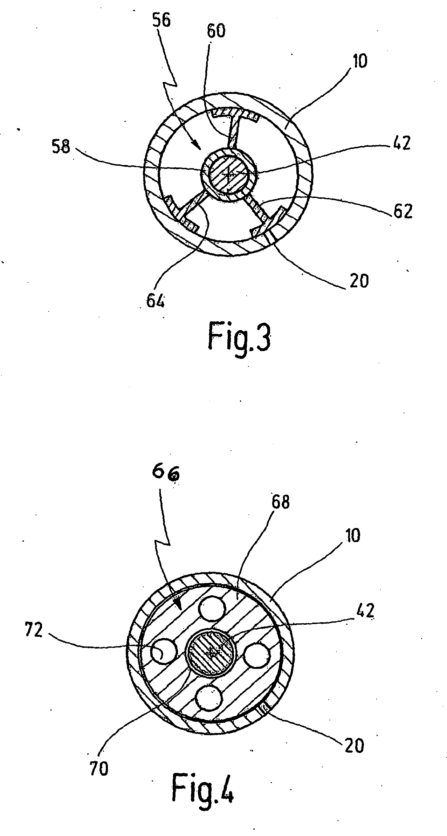

Fig. 3 zeigt einen Querschnitt durch die flexible Hohlwelle 10, und zwar in einem Bereich, in dem auf die Außenseite des Drahtes 42 ein Abstandhalter 56 aufgeschoben ist.

Dazu weist der Abstandhalter 56 eine Hülse 58 auf, deren lichter Innendurchmesser in etwa dem Außendurchmesser des Drahtes 42 entspricht, so dass der Abstandhalter 56 auf den Draht 42 aufgeschoben werden kann. Von der Hülse 58 stehen umfänglich gleichmäßig verteilt radial drei Beine 60, 62 und 64 vor, die endseitig in Anlagefüßchen, die hier nicht näher bezeichnet sind, enden. Über diese Anlagefüßchen liegen die Beine 60, 62 und 64 an der Innenseite der Wandung 18 der Hohlwelle 10 an. Dadurch wird für eine Beabstandung oder mittige Zentrierung des Drahtes 42 im Inneren der Hohlwelle 10 gesorgt. Es können mehrere axial beabstandete Abstandhalter 56 aufgeschoben werden, wobei diese im Wesentlichen im mittigen Bereich über die Länge der Hohlwelle angeordnet sind, also dort, wo die Durchbiegung am größten ist, daher ist dieser Abstandhalter 56 in der Schnittdarstellung von Fig. 2 nicht ersichtlich.

Der Abstandhalter 56 kann als Kunststoffspritzgussteil oder auch aus metallischem Material hergestellt sein.

Aus der Schnittdarstellung von Fig. 3 ist ersichtlich, dass trotz des Vorhandenseins des Abstandhalters 56 in axialer Richtung durch die Hohlwelle 10 hindurch Medien geführt werden können, sei es Spülmedien, die von proximal nach distal gefördert werden sollen, oder Medien, die über die Hohlwelle von distal nach proximal abgesaugt werden können, also beispielsweise Spülflüssigkeit, Blut, abgetrenntes Gewebe, abgeriebene Knochenteile etc. Letztendlich wird die Konstruktion so gewählt, dass ein möglichst geringer Strömungswiderstand durch die Abstandhalter 56 erzeugt wird.

In Fig. 4 ist ein weiteres Ausführungsbeispiel eines Abstandhalters 66 dargestellt, dessen Körper als Scheibe 68 ausgebildet ist, die eine mittige Öffnung 70 aufweist. Über diese mittige Öffnung 70 ist die Scheibe 68 auf den Draht 42 aufgeschoben. In der Scheibe 68 sind weitere Durchtrittsöffnungen 72 vorhanden, hier vier umfänglich gleichmäßig verteilte Durchtrittsöffnungen 72, um den Durchfluss der zuvor angesprochenen Medien zu ermöglichen.

## Patentansprüche

1. Medizinisches Instrument (28) mit einem gekrümmten Schaft (30), in dem eine flexible Hohlwelle (10) aufgenommen ist, wobei die flexible Hohlwelle (10) ein proximales Ende (14) zum Koppeln mit einem Antrieb (26) des medizinischen Instrumentes (28) und ein distales Ende (12) aufweist, an dem ein Werkzeug (32) angeordnet ist, wobei die Hohlwelle (10) eine Wandung (18) mit Einschnitten (20) aufweist, so dass die Hohlwelle auch in gekrümmtem Zustand Drehkräfte übertragen kann, **dadurch gekennzeichnet, dass** am distalen Ende (12) der Hohlwelle (10) ein Anschlag (48) vorgesehen ist, der mit einem Anschlag am Schaft (30) derart zusammenwirkt, dass ein Stauchen der Hohlwelle (10) über ein bestimmtes Maß gesperrt ist, und dass sich in der Hohlwelle (10) ein flexibler, in axialer Richtung jedoch undehnbarer und unstauchbarer Draht (42) vom distalen (12) zum proximalen Ende (14) erstreckt.

2. Medizinisches Instrument nach Anspruch 1, **dadurch gekennzeichnet, dass** der Anschlag (48) an einer drehbar am distalen Ende auf der Hohlwelle (10) angebrachten Hülse (44) ausgebildet ist.

3. Medizinisches Instrument nach Anspruch 2, **dadurch gekennzeichnet, dass** der Anschlag (48) als eine Schulter (46) ausgebildet ist.

4. Medizinisches Instrument nach Anspruch 2 oder 3, **dadurch gekennzeichnet, dass** die Hülse (44) axial beweglich am distalen Ende (12) angebracht ist.

5. Medizinisches Instrument nach einem der Ansprüche 1 bis 4, **dadurch gekennzeichnet, dass** das Werkzeug (32) am distalen Ende (12) über den Draht (42) mit dem proximalen Ende (14) verbunden ist.

6. Medizinisches Instrument nach einem der Ansprüche 1 bis 5, **dadurch gekennzeichnet, dass** die Einschnitte (20) der Hohlwelle (10) als schraubenlinienförmig gewundener Schlitz (22) ausgebildet sind.

7. Medizinisches Instrument nach Anspruch 6, **dadurch gekennzeichnet, dass** der Schlitz (22) als mäanderförmiger Schlitz ausgebildet ist.

8. Medizinisches Instrument nach einem der Ansprüche 1 bis 7, **dadurch gekennzeichnet, dass** Abstandhalter (56, 66) vorhanden sind, die den Draht (42) im Inneren der Hohlwelle (10) im Abstand von deren Wandung (18) halten.

9. Medizinisches Instrument nach Anspruch 8, **dadurch gekennzeichnet, dass** die Abstandhalter (56, 66) derart ausgestaltet sind, dass Medien im Inneren der Hohlwelle (10) in axialer Richtung transportierbar sind.

10. Medizinisches Instrument nach einem der Ansprüche 1 bis 9, **dadurch gekennzeichnet, dass** der Draht (42) und/oder die innere Wandung (18) der Hohlwelle mit einem reibungsgünstigen Material versehen sind.

## Claims

1. Medical instrument (28) having a curved shank (30) housing a flexible hollow shaft (10),
wherein the flexible hollow shaft (10) has a proximal end (14) for coupling to a drive mechanism (26) of the medical instrument (28) and a distal end (12) on which a tool (32) is arranged, wherein the hollow shaft (10) has a wall (18) with cuttings (20) such that the hollow shaft (10) can also transmit rotary forces in the curved state, **characterized in that** a limit stop (48) is provided at the distal end (12) of the hollow shaft (10) which acts with a limit stop at the shaft (30) **in that** a compression of the hollow shaft (10) beyond a certain extend is stopped, and **in that** a wire (42) extends from the distal end (12) to the proximal end (14) within the hollow shaft (10) which wire is flexible but axially inextensible and axially incompressible.

2. Medical instrument of claim 1, **characterized in that** the limit stop (48) is provided on a sleeve (44) mounted rotatable at the distal end of the hollow shaft (10).

3. Medical instrument of claim 2, **characterized in that** the limit stop (48) is designed as a shoulder (46).

4. Medical instrument of claims 2 or 3, **characterized in that** the sleeve (44) is mounted axially movable at the distal end (12).

5. Medical instrument of anyone of claims 1 through 4, **characterized in that** the tool (32) at the distal end (12) is connected to the proximal end via the wire (42).

6. Medical instrument of anyone of claims 1 through 5, **characterized in that** the cuttings (20) of the hollow shaft (10) are designed as a slit (22) which winds in the shape of a helical line.

7. Medical instrument of claim 6, **characterized in that** the slit (22) is designed as a slit with a meandering configuration.

8. Medical instrument of anyone of claims 1 through 7, **characterized in that** spacers (56, 66) are provided which keep the wire (42) inside the hollow shaft (10) at a distance from the wall (18) of the latter.

9. Medical instrument of claim 8, **characterized in that** the spacers (56, 66) are designed in such a way that media in the interior of the hollow shaft (10) can be transported in the axial direction.

10. Medical instrument of anyone of claims 1 through 9, **characterized in that** the wire (40) and/or the inside wall (18) of the hollow shaft are provided with a material that is favourable in terms of friction.

## Revendications

1. Instrument médical (28) comportant une tige courbe (30) dans laquelle un arbre creux flexible (10) est logé, ledit arbre creux flexible (10) comprenant une extrémité proximale (14) dédiée à l'accouplement à un entraînement (26) dudit instrument médical (28), et une extrémité distale (12) sur laquelle un outil (32) est disposé, l'arbre ceux (10) présentant une paroi (18) munie d'incisions (20), de telle sorte que ledit arbre creux puisse transmettre des forces de rotation, également à l'état courbé, **caractérisé par le fait qu'**une butée (48) prévue à l'extrémité distale (12) de l'arbre ceux (10) coopère avec une butée située sur la tige (30), de manière à bloquer un refoulement dudit arbre creux (10) selon une ampleur déterminée, et qu'un fil métallique (42) flexible, mais cependant non extensible et non compressible dans le sens axial, s'étend dans l'arbre creux (10) depuis l'extrémité distale (12) jusqu'à l'extrémité proximale (14).

2. Instrument médical selon la revendication 1, **caractérisé par le fait que** la butée (48) est ménagée sur une douille (44) implantée sur l'arbre creux (10) avec faculté de rotation, à l'extrémité distale.

3. Instrument médical selon la revendication 2, **caractérisé par le fait que** la butée (48) est réalisée sous la forme d'un épaulement (46).

4. Instrument médical selon la revendication 2 ou 3, **caractérisé par le fait que** la douille (44) est implantée sur l'extrémité distale (12), avec mobilité axiale.

5. Instrument médical selon l'une des revendications 1 à 4, **caractérisé par le fait que** l'outil (32), placé à l'extrémité distale (12), est relié à l'extrémité proximale (14) par l'intermédiaire du fil métallique (42).

6. Instrument médical selon l'une des revendications 1 à 5, **caractérisé par le fait que** les incisions (20) de l'arbre creux (10) sont réalisées sous la forme d'une fente (22) à spires hélicoïdales.

7. Instrument médical selon la revendication 6, **caractérisé par le fait que** la fente (22) est réalisée sous la forme d'une fente décrivant des méandres.

8. Instrument médical selon l'une des revendications 1 à 7, **caractérisé par** la présence d'organes d'espacement (56, 66) qui maintiennent le fil métallique (42), à l'intérieur de l'arbre creux (10), à distance de la paroi (18) de ce dernier.

9. Instrument médical selon la revendication 8, **caractérisé par le fait que** les organes d'espacement (56, 66) sont agencés de façon telle que des fluides puissent être convoyés, dans le sens axial, à l'intérieur de l'arbre creux (10).

10. Instrument médical selon l'une des revendications 1 à 9, **caractérisé par le fait que** le fil métallique (42), et/ou la paroi intérieure (18) de l'arbre creux, présente(nt) un matériau propice au frottement.
